# EUROPÄISCHE PATENTANMELDUNG

(11) **EP 1 088 586 A1**
(43) Veröffentlichungstag der Anmeldung: **04.04.2001**
(21) Anmeldenummer: 00120252.2
(22) Anmeldetag: 27.09.2000
(51) Int. Cl.: B01J 19/10, B01D 9/00, B01J 19/00, C07C 51/43

(54) **Verminderung der Krusetnbildung bei Kristallisationsprozessen**

(30) Priorität: 29.09.1999 DE 19946671
(71) Anmelder: BASF AKTIENGESELLSCHAFT, 67056 Ludwigshafen (DE)
(72) Erfinder: Setzer, Udo, Dr., 69493 Hirschberg (DE); Otto, Bernhard, Dr., 67117 Limburgerhof (DE); Bechtel, Siegfried, 68623 Lampertheim (DE); van Gelder, Richard, Dr., 67061 Ludwigshafen (DE); Fetzer, Thomas, Dr., 67346 Speyer (DE); Borgwart, Joachim, 67067 Ludwigshafen (DE); Rauls, Matthias, Dr., 67117 Limburgerhof (DE); Gahn, Christoph, 67346 Speyer (DE)
(74) Vertreter: Isenbruck, Günter, Dr.

(57) **Zusammenfassung**

Die Erfindung bezieht sich auf ein Verfahren und eine Vorrichtung zur Vermeidung von Krustenbildungen in Reaktoren oder Kristallisatoren (13) wie Betriebskristallisatoren für Suspensionen (11), wobei an den Wänden (6, 21) und am Boden (7) der Kristallisatoren (13) Einstrahlpunkte (4) zur Einkopplung von Ultraschallschwingungen durch Sonotroden (3) vorgesehen sind, die mit elektromechanischen Wandlern (2) in Verbindung stehen. Die Ultraschallfrequenz wird an Hochfrequenzgeneratoren (1) erzeugt.

## Beschreibung

Die Erfindung bezieht sich auf eine Verminderung der Krustenbildung von organischen und anorganischen Substanzen an den Innenwänden von Kristallisations- oder Reaktionsbehältern, wie beispielsweise Adipinsäurekrusten an den Seitenwänden großer Kristallistoren.

Die Nutzung von Impulsultraschalltechnik für Kesselsteinverhinderung an Wärmetauschern ist beispielsweise aus dem Aufsatz in der Zeitschriftenreihe 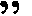 Sanitär- und Heizungstechnik 6/1999" von V. Podolyak, U. Schmucker und S. Sperling, Fraunhofer Institut, Magdeburg bekannt. Aus dem Beitrag 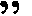 Ultraschall: kostensparend und umweltfreundlich" geht hervor, daß Impulsultraschallschwingungen zur Verhinderung von Ablagerungen an Wärmetauschern verwendet werden. Impulsultraschallapparate haben kleine Außenabmessungen und benötigen sehr wenig elektrische Energie. Die Impulsfrequenz läßt sich auf einfache Weise regulieren, wobei die Frequenz der Eigenschwingungen des Ultraschallumwandlers bei etwa 20 kHz liegt. Ein Ultraschallamplitudenverstärker wird üblicherweise an der Wandung eines vor Kesselstein, Rost zu schützenden Wärmetauschers befestigt oder es werden speziell ausgebildete Strahler direkt in die Flüssigkeit eingetaucht. Dabei müssen der richtige Punkt oder die richtigen Punkte der optimalen Übertragung der Energie gefunden werden. Die Wirksamkeit des Impulsultraschalls wird hauptsächlich durch die Wahl dieser Einstrahlpunkte bestimmt.

EP-O 584 685 A2 betrifft einen Reaktor zur Durchführung chemischer Reaktionen. Am Boden des Reaktors sind mindestens drei Sonotroden und am Mantel sind mindestens sechs Sonotroden angeordnet. Die Sonotroden lassen sich sowohl in den Boden als auch in den Mantel des Reaktors integrieren, der neben einer einwandigen Ausführungsweise auch in doppelwandiger Ausführungsform ausgeführt sein kann.

US-5,471,001 bezieht sich auf ein Kristallisationsverfahren für Adipinsäure, wobei eine wässrige Suspension, welche gelöste Adipinkristalle enthält einer Ultraschallerregung geringer Intensität ausgesetzt wird, während die wässrige Lösung gekühlt wird und/oder während der Wassergehalt der wässrigen Lösung abgesenkt wird. Die Intensität der Ultraschallschwingung liegt im Frequenzbereich zwischen 20 bis 100 kHz, wobei die wässrige Lösung durch ein in das Reaktorinnere hineinragendes Rührelement im Zeittakt von 5 bis 20 Sekunden die Sonotroden passiert.

Der Erfindung liegt die Aufgabe zugrunde, die Anhaftung von Feststoff bzw. Kristallen an den Wänden von Behältern und Kristallisatoren so stark herabzusetzen, daß das Anwachsen einer Feststoffschicht wirksam verhindert wird.

Erfindungsgemäß wird diese Aufgabe dadurch gelöst, daß bei einem Verfahren zur Vermeidung von Krustenbildung in Reaktoren oder Kristallisatoren wie zum Beispiel Betriebskristallisatoren für Suspensionen an deren Wänden und im Bodenbereich Einstrahlpunkte für die Einkopplung von Ultraschallschwingungen durch Schwingungsanreger, zum Beispiel Sonotroden vorgesehen sind, die mit elektromechanischen Wandlern in Verbindung stehen und deren Ultraschallfrequenz über einen Hochfrequenzgenerator jeweils erzeugt werden. Die Einkopplung der Ultraschallschwingungen erfolgt in der Regel im Bereich ober- und/oder unterhalb des Suspensionsspiegels des Behälterinhaltes.

Durch die Einkopplung von Ultraschallschwingungen hat sich auf überraschende Weise ergeben, daß im Bereich der Einkopplungszone die Krustenbildung vermieden werden kann. Die Einstellung der optimalen Verfahrensparameter zur Vermeidung von Krustenaufbau aus Suspensionen oder Lösungen an Behälterwänden kann über die Wahl der Ultraschallfrequenz und der Schwingungsamplitude, sowie durch die Anzahl und Position der Sonotroden und die Einwirkzeit der Schwingungen erfolgen.

Das Verfahren kann sowohl bei Batchapparaten als auch bei kontinuierlich betriebenen Apparaten eingesetzt werden. Die Temperierung der Suspensionen oder Lösungen kann über externe oder interne Wärmetauscher, über an der Behälterwand angeordnete Temperierkreisläufe oder durch die Einstellung des Druckes bei der Verdampfung des Lösungsmittels im Vakuum erfolgen.

In bevorzugter Ausgestaltung des erfindungsgemäß vorgeschlagenen Verfahrens kann die Schwingungsfrequenz, die die Schwingungsüberträger (Sonotroden) an ihren jeweiligen Einstrahlpunkten in den Kristallisator oder einen Behälter aufweisen, im Bereich zwischen 16 kHz und 100 kHz liegen. Es sind aber auch Schwingungserregungen außerhalb des Ultraschallbereiches, zum Beispiel mit Frequenzen im Bereich zwischen 100 Hz bis 16kHz möglich. Die Einstrahlzone für die Ultraschallschwingungen ist in vorteilhafter Weise durch ein Mantelsegment des Kristallisators gebildet. So kann ein gleichmäßiges Aufprägen von Ultraschallschwingungen auf die Suspension erfolgen, so daß insbesondere der durch die Suspension benetzte Randbereich des Kristallisators oder des Behälters mit Ultraschallschwingungen beaufschlagt ist. Daher sind die Sonotroden insbesondere ringförmig um den Kristallisator herum angeordnet; sie lassen sich ferner besonders vorteilhaft in mehreren ringförmig übereinander angeordneten Ebenen anordnen. Dabei ist darauf zu achten, daß der Abstand zwischen den Sonotroden bzw. zwischen Sonotrodenpaaren ein nicht ganzzahliges Vielfaches der halben Wellenlänge, gebildet aus der Erregerfrequenz und der Schallgeschwindigkeit des Behälter- oder Reaktormaterials, beträgt. Dies dient der Vermeidung ortsfester Knoten an Behälter- oder Reaktorwand.

Weiterhin können die Schwingungserreger auch taktweise betrieben werden, das heißt die Schwingungserregung erfolgt in gewissen Zeitabständen mit einer gewissen Periodenlänge, wobei entweder jeweils nur ein Schwingungserreger oder mehrere gleichzeitig arbeiten. Die Amplituden der Schwingungserreger liegen vorteilhafterweise im Bereich zwischen 0,1 bis 100 µm. Im Falle von Ultraschallschwingungsanregung liegen die Leistungsaufnahmen am Hochfrequenzgenerator pro Sonotrode im Bereich zwischen 100 und 10000 Watt.

In der ebenfalls offenbarten Vorrichtung zur Vermeidung von Krustenbildung an Kristallisatoren für Suspensionen sind an den Kristallisatorwänden jeweils Einstrahlpunkte für von Sonotroden aufgeprägte Ultraschallschwingungen vorgesehen, wobei die Ultraschallfrequenz an Hochfrequenzgeneratoren erzeugt wird und in einem Bereich zwischen dem Suspensionsspiegel im Kristallisator oder im Behälter ringförmig den Kristallisator oder den Behälter umgebende Sonotroden angeordnet sind.

Anhand einer Zeichnung wird die Erfindung nachstehend näher erläutert.

Es zeigt:
- Figur 1: einen Laboraufbau mit einem Behälter für eine Suspension mit nach oben offener Ausdampfzone und
- Figur 2: einen geschlossenen Kristallisator mit gekapselter Ausdampfzone

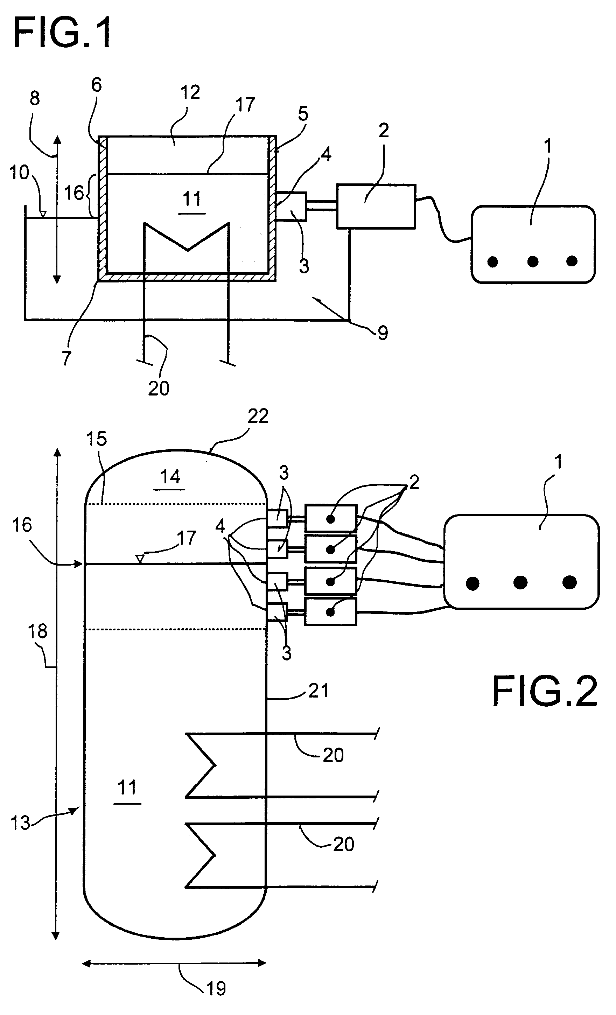

Fig. 1 zeigt den Aufbau eines Laborversuches mit einer Behälterkonfiguration mit zur Atmosphäre offener Ausdampfzone. In dieser Konfiguration wird eine Frequenz für an einem Einstrahlpunkt 4 aufzubringende Ultraschallschwingungen an einem Hochfrequenzgenerator 1 erzeugt. Mittels eines zwischen Sonotrode 3 und Hochfrequenzgenerator 1 zwischengeschalteten, elektromechanischen Wandlers 2 werden die Sonotroden 3 mit Hochfrequenz beaufschlagt, die dann auf die Außenfläche 5 der Behälterwand 6 einwirkt und diese zu hochfrequenten Schwingungen anregt. Eine Suspension 11 ist von Behälterwand 6 und Behälterboden 7 umschlossen, wohingegen die oberhalb des Suspensionsspiegels 17 sich einstellende Ausdampfzone 12 zur Umgebung hin offen ist.

Die im Behälter befindliche Suspension kann beispielsweise Adipinsäure sein auch Hexadinsäure bezeichnet, HOOC-(CH₂)₄-COOH; C₆H₁₀O₄ die farblos ist, einen Schmelzpunkt von 153° sowie einen Siedepunkt von 265°C bei 166 hPa aufweist. Sie ist in Wasser schwer löslich (Verhältnis 1 : 60). Adipinsäure wird durch Oxidation von Fett mit Salpetersäure erhalten und entsteht auch bei der Oxidation von Cyclohexanreichen Erdölen. Adipinsäure kann auch durch Oxidation von Cyclohexanol oder Cyclohexanon mittels HNO₃ oder Luft gewonnen werden; über eine Einstufenoxidation von Cyclohexan ist Adipinsäure ein wichtiger Rohstoff für die Herstellung von Nylon, ferner ist Adipinsäuredinitril ein wichtiges Zwischenprodukt bei der Herstellung von Weichmachern insbesondere DIDA und DOA.

Im in Fig. 1 dargestellten Beispiel wird die Außenfläche 5 der Behälterwand 6 durch eine Sonotrode 3 beaufschlagt. Bei einer ringförmigen Anordnung von Sonotroden 3 entlang der Außenfläche 5 in Umfangsrichtung läßt sich ein Bereich 16 als Einstrahlzone für Ultraschallschwingungen in die Suspension 11 erzielen. Die Einstrahlzone 16 ergibt sich entlang eines Mantelsegmentes des Behälters 6 durch die Lage des Suspensionsspiegels 17 im Inneren des Behälters 6. Die Sonotroden 3 lassen sich im Bereich der Einstrahlpunkte 4 leicht an der Außenfläche 5 des Behälters 6 befestigen.

Fig. 2 zeigt einen Kristallisator 13, der eine Wandstärke 21 von 10 mm und ein Fassungsvermögen von ca. 50 m³ aufweisen kann.

Der Kristallisator 13 ist mit einer Haube 22 versehen, so daß sich eine geschlossene Ausdampfzone 14 ergibt. Der Einstrahlbereich 16, in dem die Einstrahlpunkte 4 der Sonotroden 6 liegen, ist als Mantelsegment des Kristallisators 13 ausgebildet und durch zwei gestrichelte Linien angedeutet. Der Kristallisator 13 hat eine Höhe 18 von etwa 10 m und einen Durchmesser 19 von 3 bis 4 m. Im in Fig. 2 dargestellten Ausführungsbeispiel sind im Bereich der Einstrahlzone 16 mehrere übereinanderliegende Sonotroden 3 dargestellt, die jeweils über einen elektromechanischen Wandler 2, der mit einem Hochfrequenzgenerator in Verbindung steht, angesteuert werden. Im hier dargestellten Ausführungsbeispiel sind die Sonotroden 3 auf nur einer Seite des Kristallisators 13 angeordnet, sie können diesen jedoch auch in mehreren Ebenen ringförmig umschließen, so daß die gesamte Einstrahlzone 16 durch Ultraschallschwingungen über angeschlossene Sonotroden an deren Einstrahlpunkten 4 beaufschlagt werden kann. In Sonderfällen ist auch die Schwingungsanregung im Bodenbereich möglich. Die Temperierung kann über externe und/oder interne Wärmetauscher bzw. am Behälter angebrachte Temperiermittel oder durch Verdampfung des Lösemittels über ein angelegtes Vakuum erfolgen.

Im hier dargestellten Ausführungsbeispiel sind im unteren Teil des Kristallisators 13 zwei Heizelemente 20 schematisch angedeutet, mit denen eine Temperierung der im Kristallisator 13 aufgenommenen Suspension 11 erfolgen kann. Neben den hier dargestellten Heizelementen 20, die in das Innere des Kristallisators 13 hineinreichen, können ebensogut Kühlmittelschlangen in das Innere des Kristallisators 13 reichen, mit denen eine Abkühlung der Suspension 11 erreicht werden kann. Der Kristallisator 13 kann, auch wie in Fig. 1 in kleinerem Maßstab dargestellt, von einem Kühlmedium umgeben sein oder auch zusätzlich einen externen Wärmetauscher enthalten.

Mittels des erfindungsgemäßen Verfahrens und der erfindungsgemäß offenbarten Vorrichtung lassen sich neben Adipinsäure in einem Kristallisator 13, der hier ohne interne und externe Kühlung dargestellt ist, auch andere Suspensionen verarbeiten, ohne daß sich an den Innenseiten der Kristallisatorwände 21 Verkrustungen ausbilden können. Durch entsprechende Wahl der Sonotrodenanordnung an der Außenseite der Kristallisatorwand 21 läßt sich der mantelförmige Einstrahlbereich 16 in seiner Geometrie verändern, so daß unterschiedlichem Verkrustungsverhalten Rechnung getragen werden kann und sich durch Vorwahl bestimmter Frequenzen und Amplituden am Hochfrequenzgenerator 1 anwendungsspezifisch größere oder kleinere verkrustungsfreie Zonen ausbilden lassen.

### Bezugszeichenliste

- 1: Hochfrequenzgenerator
- 2: elektromechanischer Wandler
- 3: Sonotrode
- 4: Einstrahlfläche
- 5: Außenfläche
- 6: Behälterwand
- 7: Behälterboden
- 8: Behälterhöhe
- 9: Wasserbad
- 10: Wasserstand
- 11: Suspension
- 12: offene Ausdampfzone
- 13: Betriebskristallisator
- 14: geschlossene Ausdampfzone
- 15: Zylindersegment
- 16: Einstrahlzone
- 17: Suspensionsspiegel
- 18: Kristallisatorhöhe
- 19: Durchmesser
- 20: Heizelement
- 21: Kristallisatorwand
- 22: Kristallisatorhaube

## Patentansprüche

1. Verfahren zur Vermeidung von Krustenbildung in Reaktoren oder Kristallisatoren (13) wie Betriebskristallisatoren für Lösungen und Suspensionen (11), wobei an den Wänden (6, 21) und am Boden (7) der Behälter (13) Einstrahlpunkte (4) für die Einkopplung von Ultraschallschwingungen durch Sonotroden (3) vorgesehen sind, die mit elektromechanischen Wandlern (2) in Verbindung stehen und deren Ultraschallfrequenz an einem Hochfrequenzgenerator (1) erzeugt wird, dadurch gekennzeichnet, daß in einem Bereich (16) um den Flüssigkeits- bzw. Suspensionsspiegel (17) innerhalb des Behälters (13) Ultraschallschwingungen (4) eingekoppelt werden.

2. Verfahren gemäß Anspruch 1, dadurch gekennzeichnet, daß die Ultraschallfrequenz der Sonotroden (3) zwischen 16 kHz und 20 kHz liegt und die Amplituden der Erregerschwingung zwischen 0,1 bis 100 µm liegt.

3. Verfahren gemäß Anspruch 1, dadurch gekennzeichnet, daß die Schwingungsfrequenz der Schwingungserreger (3) außerhalb des Ultraschallbereiches im Frequenzbereich zwischen 100 Hz und 16 kHz liegt.

4. Verfahren gemäß Anspruch 1, dadurch gekennzeichnet, daß die Schwingungserreger (3) kontinuierlich betrieben werden.

5. Verfahren gemäß Anspruch 1, dadurch gekennzeichnet, daß die Schwingungserreger (3) taktweise einzeln bzw. mehrere gleichzeitig und in bestimmten Zeitabständen und mit bestimmten Perioden betrieben werden.

6. Verfahren gemäß Anspruch 1, dadurch gekennzeichnet, daß der Abstand zwischen den Schwingungserregern (3) bzw. zwischen Paaren von Schwingungserregern (3) ein nicht ganzzahliges Vielfaches der halben Wellenlänge, gebildet aus der Erregerfrequenz und der Schallgeschwindigkeit des Behältermaterials, beträgt.

7. Verfahren gemäß Anspruch 1, dadurch gekennzeichnet, daß der die Einstrahlzone für die Ultraschallschwingungen bildende Bereich (16) ein Mantelsegment des Kristallisators (13) ist.

8. Verfahren gemäß Anspruch 1, dadurch gekennzeichnet, daß der die Einstrahlzone für die Ultraschallschwingungen bildende Bereich (16) der Boden (7) des Behälters (13) ist.

9. Verfahren gemäß Anspruch 7, dadurch gekennzeichnet, daß bei Einkopplung von Ultraschallschwingungen in der Einstrahlzone (16) durch ringförmig die Zone (16) umgebende Sonotroden (3) an der Kristallisatorwand (21) erfolgt.

10. Verfahren gemäß Anspruch 1, dadurch gekennzeichnet, daß die Temperierung des Behälters (6, 21) durch ein diesen umgebendes Kühlmedium (9) erfolgt.

11. Verfahren gemäß Anspruch 1, dadurch gekennzeichnet, daß die Temperierung des Behälters (6, 21) durch ein einen separaten Kreislauf durchströmendes Kühlmedium erfolgt.

12. Vorrichtung zur Vermeidung von Krustenbildung an Kristallisatoren (13), wie Betriebskristallisatoren für Suspensionen (11), wobei an den Wänden (6, 21) und am Boden (7) des Behälters (13) Einstrahlpunkte (4) zur Einkopplung von Ultraschallschwingungen durch Sonotroden (3) vorgesehen sind, die mit elektromechanischen Wandlern (2) verbunden sind, wobei die Ultraschallfrequenz an Hochfrequenzgeneratoren (1) vorgebbar ist, dadurch gekennzeichnet, daß in einem Bereich (16) zwischen dem Suspensionspegel (17) im Kristallisator (13) und der Einflußzone eines Kühlmediums (9) ringförmig den Kristallisator (13) umgebende Sonotroden (3) angeordnet sind.

13. Vorrichtung gemäß Anspruch 12, dadurch gekennzeichnet, daß der Bereich (16) ein Mantelsegment eines Kristallisators (13) darstellt.

14. Vorrichtung gemäß Anspruch 12, dadurch gekennzeichnet, daß zur Abkühlung der Lösung oder der Suspension (11) ein von außen einwirkendes Kühlmediums (9) oder ein die Lösung oder die Suspension (11) im Kristallisator (13) in separatem Kreislauf durchströmendes Kühlmedium vorgesehen ist oder die Kühlung infolge Verdampfung des Lösemittels durch ein angelegtes Vakuum erfolgt.
